(19)

Europäisches
Patentamt
European
Patent Office
Office européen
des brevets

(11) **EP 3 132 797 A1**

(12) **EUROPEAN PATENT APPLICATION**
published in accordance with Art. 153(4) EPC

(43) Date of publication:
**22.02.2017 Bulletin 2017/08**

(21) Application number: **14906760.5**

(22) Date of filing: **25.11.2014**

(51) Int Cl.:
***A61K 33/26*** (2006.01)          ***A61P 3/10*** (2006.01)
***A61K 9/08*** (2006.01)          ***A61K 9/48*** (2006.01)

(86) International application number:
**PCT/CN2014/092146**

(87) International publication number:
**WO 2016/082098 (02.06.2016 Gazette 2016/22)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Profeat Biotechnology Co. Ltd.
Taoyuan City 33045 (TW)**

(72) Inventors:
• **LIN, Tsun-Yuan
Taoyuan City
Taoyuan County (TW)**
• **JAN, Hsun-Jin
Taoyuan City
Taoyuan County (TW)**

• **FU, Chai-Hui
Taoyuan City
Taoyuan County (TW)**
• **CHEN, Tsang-Tse
Taoyuan City
Taoyuan County (TW)**
• **CHEN, Mu-Kuei
Taoyuan City
Taoyuan County (TW)**
• **LEE, Horng-Mo
Taoyuan City
Taoyuan County (TW)**

(74) Representative: **Becker Kurig Straus
Patentanwälte
Bavariastrasse 7
80336 München (DE)**

(54) **USE OF COMPOSITION CONTAINING IRON (II) AMINO ACID CHELATE IN PREPARATION OF DRUG FOR AMELIORATING DIABETES**

(57)     Provided is a use of a composition containing a ferrous amino acid chelate for the preparation of pharmaceuticals used in diabetes improvement. The pharmaceuticals comprise an effective amount of the composition containing the ferrous amino acid chelate and pharmaceutically acceptable carriers to improve diabetes. The amino acid is able to be chelated with the ferrous iron in a chelated state stably as passing through a stomach. The composition containing the ferrous amino acid chelate can effectively lower blood glucose and improve insulin sensitivity. The pharmaceuticals prepared by the composition containing the ferrous amino acid chelate have effect on diabetes improvement.

EP 3 132 797 A1

**Description**

The present invention relates to a use of a composition containing a ferrous amino acid chelate, especially to a use of the composition for the preparation of pharmaceuticals used in diabetes improvement.

Metabolic syndrome is a disease of civilization caused by lifestyle habits and dietary habits of modern man (Esposito *et al.,* 2007). According to the definition defined by the World Health Organization (WHO) in 1998, a person who has a syndrome of impaired glucose tolerance or insulin resistance, and additional two syndromes of hypertension, obesity, dyslipidemia or microalbuminuria can be diagnosed as suffering the metabolic syndrome. In Taiwan, a person who has three of the following five conditions is diagnosed as having metabolic syndrome. The five conditions include (1) the waist circumference of male is greater than or equal to 90 cm, and the waist circumference of female is greater than or equal to 80 cm; (2) the triacylglycerol is greater than 150 mg/dl; (3) the high-density lipoprotein (HDL) of male is less than 40 mg/dl, and the HDL of female is less than 50 mg/dl; (4) the systolic blood pressure is higher than 130 mmHg, and the diastolic blood pressure is higher than 85 mmHg, and (5) the value of fasting blood glucose is greater than 110 mg/dl. The rate of suffering from metabolic syndrome of Taiwanese is increasing in accordance with increasing age, and among top ten causes of death, many diseases are related to the metabolic syndrome. The average life expectancy of patients with metabolic syndrome is shorter than normal person. The reason is that the cardiovascular disease caused by high blood pressure or hyperlipidemia, or the diabetes caused by insulin resistance will cause acute complications.

There are two main types of diabetes, type 1 diabetes and type 2 diabetes. The type 1 diabetes is usually diagnosed in young adults or children, and only 5% of people with diabetes suffer type 1 diabetes. A person with the type 1 diabetes cannot produce insulin by himself, and the person needs lifelong insulin therapy. The type 2 diabetes is usually diagnosed in patients over 40 ages, and 95% of people with diabetes suffer type 2 diabetes. The type 2 diabetes is caused by impairments of insulin signaling, leading cells be incapable of glucose uptake. Therefore, patients with type 2 diabetes will keep feeding and cause high blood pressures. The high concentration of glucose in the blood will stimulate islet cells to secrete insulin and cause hyperinsulinism, and finally generate insulin resistance and cause pancreas failure. Parts of the patients with type 2 diabetes need drugs or insulin therapy. Some acute complications such as cardiovascular disease (Vlassara, 1996), chronic renal failure (Monnier *et al.,* 1992), retinopathy (Yamagishi et al., 2002), neuropathy and microangiopathy will be caused once the diabetes is not controlled well. Microangiopathy may cause lower limb gangrene and lead patients to be amputated.

Drugs combined with diet control are commonly used as treatment for type 2 diabetes (Granberry and Fonseca, 2005). The common drugs include thiazolidinedione and metformin. Metformin is a kind of oral hypoglycemic drug with biguanide, and the mechanism is unclear. However, it is known that the metformin is able to inhibit hepatic gluconeogenesis and increase glucose uptake in muscle. The side effects caused by the metformin include diarrhea and nausea.

For the shortcomings of the side effects caused by treating type 2 diabetes with conventional chemical pharmaceuticals, the objective of the present invention is to provide a use of a composition containing a ferrous amino acid chelate for the preparation of pharmaceuticals used in improvement of diabetes. The composition containing the ferrous amino acid chelate has an effect on improvement of diabetes.

To achieve the above objective, the objective of the invention is to provide the use of a composition containing a ferrous amino acid chelate for preparation of pharmaceuticals used in diabetes improvement, wherein pharmaceuticals comprise an effective amount of the composition containing the ferrous amino acid chelate and pharmaceutically acceptable carriers.

According to the present invention, the term "the composition containing the ferrous amino acid chelate" refers to made by mixing an inorganic iron with an amino acid.

Preferably, the inorganic iron comprises, but is not limited to ferrous sulfate, ferrous chloride, or ferrous pyrophosphate, and the amino acid is glycine.

More preferably, the composition containing the ferrous amino acid chelate comprises 95% to 100% weight percentage of the ferrous glycine chelate. Furthermore preferably, the composition containing the ferrous amino acid chelate comprises 98% to 99.9% weight percentage of the ferrous glycine chelate.

Preferably, the composition containing the ferrous amino acid chelate is prepared from mixing ferrous sulfate with glycine followed by heating between 60°C and 90°C for 8 hours to 48 hours, wherein a weight ratio of the ferrous sulfate to the glycine of ferrous amino acid chelate is between 1:1.2 and 1:1.5.

The composition containing the ferrous amino acid chelate in accordance with the present invention comprises at least one ferrous amino acid chelate, and the chelating ratio of the ferrous iron to the amino acid of the composition containing the ferrous amino acid chelate is between 1:1 and 1:4. More preferably, the chelating ratio of the ferrous iron to the amino acid of ferrous amino acid chelate is between 1:1.5 and 1:2.5.

Preferably, the composition containing the ferrous amino acid chelate comprises a reducing agent. The reducing agent can maintain the oxidation state of the ferrous iron of the ferrous amino acid chelate contained in the composition. Besides, the reducing agent can also enhance the intestinal absorption rate of the composition containing the ferrous amino acid chelate in subjects. The reducing agent comprises, but is not limited to ascorbic acid, citric acid, acetic acid, propionic acid, butyric acid, lactic acid, malic acid, sulfonic acid or succinic acid.

The diabetes improvement in accordance with the present invention means effective treatment or relieving diabetes. As shown in the embodiment of the present invention, the symptoms of diabetes improvement comprises, but is not limited to lowering blood glucose, improving glucose tolerance, and enhancing insulin sensitivity.

The effective amount in accordance with the present invention means dosage of the pharmaceuticals used for effective improvement of diabetes in the required period. As shown in the embodiment of the present invention, the dosage of the pharmaceuticals used for effective improvement of diabetes can be determined by administering the composition containing the ferrous amino acid chelate in specific amount, and measuring the blood glucose, fasting blood glucose and insulin variances in a specific period of time.

Preferably, the effective amount of the composition containing the ferrous amino acid chelate is between 0.1 mg/kg/day and 15 mg/kg/day. More preferably, the effective amount is between 0.16 mg/kg/day and 12 mg/kg/day.

The pharmaceutically acceptable carriers in accordance with the present invention comprises any of physiologically compatible solvents, dispersed medium, coating materials, antibacterial agents, antifungal agents, isotonic agents, and absorption delaying and analogues thereof. The pharmaceutically acceptable carriers comprise water, saline, phosphate buffered solution, dextrose, glycerol, ethanol, analogues thereof or any combination thereof. In many conditions, preferably, the pharmaceutically acceptable carriers comprise isotonic agents, for example, sugars, polyalcohols such as mannitol and sorbitol, or sodium chloride. The pharmaceutically acceptable carriers can further comprise micro-auxiliary substances such as wetting agent, emulsifier, preservative or buffering agent.

The pharmaceuticals in accordance with the present invention comprise various dosage forms, and the dosage form comprises, but is not limited to liquid, semi-solid and solid. The liquid comprises, but is not limited to dispersion or suspension. The semi-solid and the solid comprise, but is not limited to tablet, pill, powder, liposome or suppository. The preferred dosage form of the drug is dependent on the expected mode of administration and therapeutic application.

Preferably, the dosage form of the pharmaceuticals in accordance with the present invention is for oral dosage administration or injection. The preferred mode of administration is the mode of enteral administration, such as oral administration. As shown in the embodiment of the present invention, the pharmaceuticals comprising the composition containing the ferrous amino acid chelate for effective improvement of diabetes and obese is orally administered.

Preferably, the pharmaceuticals further comprise an excipient, allowing the pharmaceuticals to be made in the dosage form applicable to enteral administration or parenteral administration.

Preferably, the dosage form of the drug for enteral administration is oral dosage form. The oral dosage form comprises, but is not limited to solution, suspension, tablet or capsule.

The composition containing the ferrous amino acid chelate in accordance with the present invention has greater effect on diabetes improvement than use of commercially available Metformin. Besides, for the reason that the molecular weight of the amino acid is small enough to be chelated with the ferrous iron in a chelating state stably as passing through a stomach of a subject. The composition containing the ferrous amino acid chelate can effectively lower blood glucose and improve insulin sensitivity.

**In the drawings:**

Fig. 1 is a bar chart of fasting blood glucose assay of obese mice fed with high fat diet in accordance with the present invention;
Fig. 2 is a curve chart of oral glucose tolerance assay of diabetic mice fed with a composition A1 in accordance with the present invention, and Met is Metformin; and
Fig. 3 is a bar chart of the area of the curve chart of oral glucose tolerance assay in Fig. 2.

**Preparation 1: Preparation of the composition containing a ferrous amino acid chelate**

The methods for preparing a composition containing a ferrous amino acid chelate were shown as follows. First, ferrous sulfate was mixed with glycine (above 98% purity) in a weight ratio of 1:1.3 followed by heating from 60°C to 90°C for 8 hours to 48 hours to form the composition containing the ferrous amino acid chelate. The chelating ratio of ferrous iron to amino acid of the ferrous amino acid chelate was between 1:1 and 1:4. The composition containing the ferrous amino acid chelate was prepared in concentrations of 0.16 $\mu$g/ml, 0.4 $\mu$g/ml, 1.2 $\mu$g/ml, 4 $\mu$g/ml, and 12 $\mu$g/ml. The composition containing the ferrous amino acid chelate was named as composition A1.

Preparation 2: Animal study of high fat diet-induced obesity mice

Table 1

|  | Control group | Comparison group | Experiment group 1 | Experiment group 2 |
|---|---|---|---|---|
| Administered with the composition A1 | - | - | 0.16 mg/kg | 0.4 mg/kg |
| Number of mice | 10 | 5 | 5 | 5 |
| Diet | Normal | High fat diet | High fat diet | High fat diet |

C57BL/6JNR male mice at 12 weeks of age (purchased from National Laboratory Animal Center) were fed under 12/12-hour light-dark cycle and supplied with water. The mice were grouped as shown in Table 1. The mice of comparison group, experiment group 1 and experiment group 2 were fed with high fat diet, and the mice of experiment group 1 and experiment group 2 were orally administered with the composition A1 at dosage of 0.16 mg/kg/day and 0.4 mg/kg/day, respectively for 3 months. The weights of the mice were measured every 3 days, and the blood glucose values of the mice were measured every 4 weeks after administering with the composition A1.

**Preparation 3: Animal study of drug-induced diabetic mice**

C57BL/6JNR male mice at 6 weeks of age (purchased from National Laboratory Animal Center) were fed with normal diet for 1 week. Each of the mice was injected intraperitoneally with 240 mg/kg nicotinamide, and after 15 minutes, each of the mice was injected intraperitoneally with 100 mg/kg streptozotocin. Two days later, each of the mice was injected with nicotinamide and streptozotocin with the same dosages and methods as shown above, followed by feeding with the high fat diet (60% fat). Two months later, the mice having fasting blood glucose higher than 140 mg/dl and impaired glucose tolerance (which meant that the blood glucose values were not measured within normal range after injecting glucose for 2 hours) were picked to be type 2 diabetes mice.

The mice were divided into 4 groups which included a control group (supplied with phosphate solution), a metformin (Met) group, a composition A1 with low dose (4 mg/kg/day) group, and a composition A1 with high dose (12 mg/kg/day) group. The weights of the mice were measured every 3 days, and the blood glucose values, oral glucose tolerance assay (OGTT), glycated hemoglobin (HbA1c) and insulin variances of the mice were measured every 4 weeks.

The blood glucose values were measured with a Chemistry Analyzer (Hitachi, Ltd., Japan). The glycated hemoglobin (hemoblobin A1c, HbA1c) was measured with a Glycohemoglobin Analyzer (Tosoh, Ltd., USA). The insulin variance was measured with enzyme-linked immunosorbent assay (ELISA) (Mercodia, Ltd. Sweden).

**Example 1: Examination of blood glucose values, glucose tolerance and insulin sensitivity of diabetic mice administered with the composition A1**

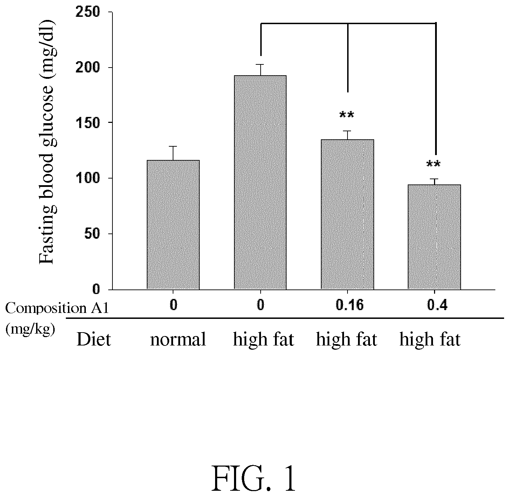

The value of blood glucose was measured according to the methods recited in the Preparation 2. With reference to Fig. 1, after administrating for 3 months, results showed that values of the fasting blood glucose of experiment group 1 (administered with the 0.16 mg/kg/day composition A1) and experiment group 2 (administered with the 0.4 mg/kg/day composition A1) were lower than that of comparison group (without administered with any composition A1) despite that the mice of experiment group 1 and experiment group 2 were fed with high fat diet. The values of the fasting blood glucose of experiment group 2 (administered with the 0.4 mg/kg/day composition A1) were lower than that of control group (fed with normal diet, without any high fat diet). Therefore, blood glucose values of the diet-induced obesity mice could be lowered by administering the composition A1.

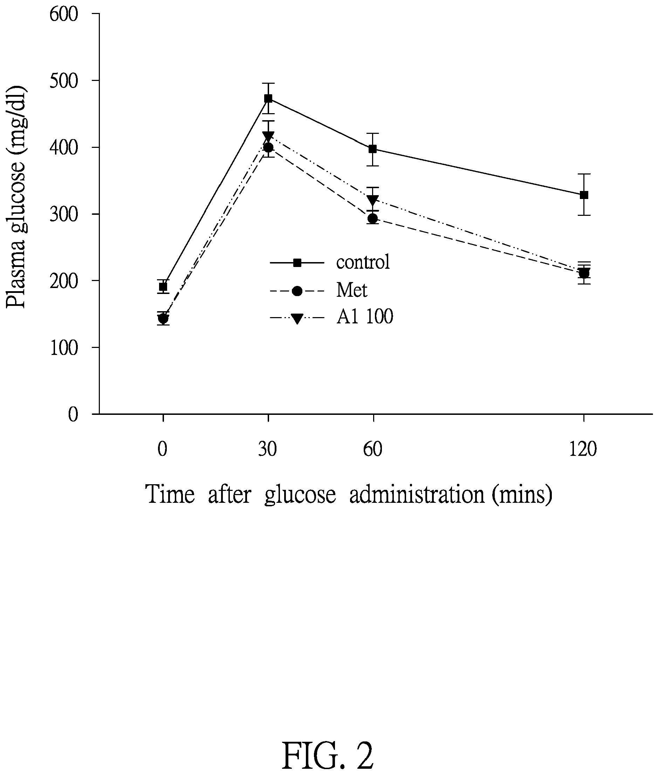

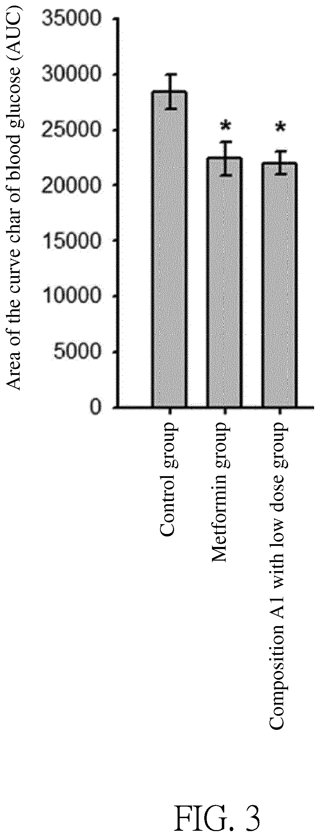

The value of blood glucose was measured according to the methods recited in the Preparation 3. With reference to Fig. 2, results showed that glucose tolerance of the metformin group and the composition A1 with high dose group or low dose group were all improved. With reference to Fig. 3, after calculating the area of the curve chart of glucose tolerance assay in Fig. 2, results showed that the composition A1 with low dose could provide greater effect than the metformin with high dose.

Table 2 Glycated hemoglobin, values of fasting blood glucose, and insulin variances of the diabetic mice

|  | Glycated hemoglobin (HbA1c) | insulin |
|---|---|---|
| Control group | 4.00±0.16 | 18.34±6.68 |
| Metformin group | 3.57±0.04* | 11.11±6.47 |
| Composition A1 with low dose group | 3.70±0.03* | 6.00±2.22* |

(continued)

|  | Glycated hemoglobin (HbA1c) | insulin |
|---|---|---|
| Glycine | 3.71±0.03* | 12.47±2.36 |
| Normal value | 2.9±0.05 | |

Table 3 Glycated hemoglobin, values of fasting blood glucose, and HOMA-IR value of the diabetic mice

|  | Fasting blood glucose | HOMA-IR |
|---|---|---|
| Control group | 203.00±17.43 | 8.59±2.00 |
| Metformin group | 169.00±10.77* | 4.53±1.42* |
| Composition A1 with low dose group | 152.71±4.74* | 2.17±0.87* |
| Composition A1 with high dose group | 166.13±5.02* | 3.85±2.08* |
| Glycine | 182.88±11.20 | 5.23±1.73 |
| Normal value | 143.5±9.14 | |

With references to Table 2 and Table 3, after administering the pharmaceuticals for one month, the value of glycated hemoglobin in each of the groups was decreased obviously. Composition A1 with low dose had the best therapeutic effect on insulin secretion and fasting blood glucose.

The index of homeostasis model assessment for insulin resistance (HOMA-IR) was calculated from the following formula:

$$\text{HOMA-IR index} = \text{insulin } (\mu U/ml) \times \text{glucose } (mmol/L)/22.5.$$

Results showed that insulin resistance effects of the metformin group and the composition A1 with low dose group were best, and HOMA-IR value of the composition A1 with low dose group was decreased from 8.59 to 2.17, which was better than 4.53 of the metformin group. Therefore, the composition A1 with low dose group had greater effects on fasting blood glucose, insulin, and HOMA-IR than the metformin group. Besides, the dosage of the composition A1 group was lower than that of the metformin group. Therefore, the composition A1 with low dose had the best effect on improvement of diabetes.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A use of a composition containing a ferrous amino acid chelate for the preparation of pharmaceuticals used in diabetes improvement, wherein the pharmaceuticals comprising an effective amount of the composition containing the ferrous amino acid chelate and pharmaceutically acceptable carriers.

2. The use as claimed in claim 1, wherein the chelating ratio of ferrous iron to the amino acid of the composition containing the ferrous amino acid chelate is between 1:1 and 1:4.

3. The use as claimed in claim 1, wherein the chelating ratio of ferrous iron to the amino acid of the composition containing the ferrous amino acid chelate is between 1:1.5 and 1:2.5.

4. The use as claimed in claim 1, wherein the effective amount of the composition containing the ferrous amino acid chelate is between 0.1 mg/kg/day and 15 mg/kg/day.

5. The use as claimed in claim 1, wherein the effective amount of the composition containing the ferrous amino acid chelate is between 0.16 mg/kg/day and 12 mg/kg/day.

6. The use as claimed in any one of claims 1 to 5, wherein the composition containing the ferrous amino acid chelate is prepared from mixing an inorganic iron with an amino acid followed by heating between 60°C and 90°C for 8 hours to 48 hours, wherein the chelating ratio of the inorganic iron to the amino acid is between 1:1.2 and 1:1.5.

7. The use as claimed in claim 6, wherein the inorganic iron is ferrous sulfate, ferrous chloride, or ferrous pyrophosphate, wherein the amino acid is glycine.

8. The use as claimed in claim 6, wherein the composition containing the ferrous amino acid chelate comprises a reducing agent, wherein the reducing agent comprises ascorbic acid, citric acid, acetic acid, propionic acid, butyric acid, lactic acid, malic acid, sulfonic acid or succinic acid.

9. The use as claimed in claim 1, wherein a dosage form of the drug is for enteral administration or parenteral administration.

10. The use as claimed in claim 9, wherein the dosage form of the drug for enteral administration is oral dosage form, wherein the oral dosage form comprises solution, suspension, tablet or capsule.

FIG. 1

FIG. 2

FIG. 3

# INTERNATIONAL SEARCH REPORT

| International application No. |
| --- |
| PCT/CN2014/092146 |

### A. CLASSIFICATION OF SUBJECT MATTER

A61K 33/26 (2006.01) i; A61P 3/10 (2006.01) i; A61K 9/08 (2006.01) i; A61K 9/48 (2006.01) i
According to International Patent Classification (IPC) or to both national classification and IPC

### B. FIELDS SEARCHED

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

WPI, EPODOC, CNPAT, CNKI, REGISTRY, CA: ferro+, iron, glycine, ferrous amino acid chelate, diabete, insulin etc.

### C. DOCUMENTS CONSIDERED TO BE RELEVANT

| Category* | Citation of document, with indication, where appropriate, of the relevant passages | Relevant to claim No. |
| --- | --- | --- |
| X | CN 102164596 A (SBI5 ALA PROMO CO., LTD.) 24 August 2011 (24.08.2011) see claims 1-4, description, paragraphs [0036], [0046], [0047] and [0058]-[0063]. | 1-10 |

☐ Further documents are listed in the continuation of Box C.          ☒ See patent family annex.

| | |
| --- | --- |
| \* Special categories of cited documents:<br><br>"A" document defining the general state of the art which is not considered to be of particular relevance<br><br>"E" earlier application or patent but published on or after the international filing date<br><br>"L" document which may throw doubts on priority claim(s) or which is cited to establish the publication date of another citation or other special reason (as specified)<br><br>"O" document referring to an oral disclosure, use, exhibition or other means<br><br>"P" document published prior to the international filing date but later than the priority date claimed | "T" later document published after the international filing date or priority date and not in conflict with the application but cited to understand the principle or theory underlying the invention<br><br>"X" document of particular relevance; the claimed invention cannot be considered novel or cannot be considered to involve an inventive step when the document is taken alone<br><br>"Y" document of particular relevance; the claimed invention cannot be considered to involve an inventive step when the document is combined with one or more other such documents, such combination being obvious to a person skilled in the art<br><br>"&" document member of the same patent family |
| Date of the actual completion of the international search<br><br>02 August 2015 | Date of mailing of the international search report<br><br>01 August 2015 |
| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br><br>XIAO, Ying<br><br>Telephone No. (86-10) 62089320 |

Form PCT/ISA /210 (second sheet) (July 2009)

## INTERNATIONAL SEARCH REPORT
Information on patent family members

International application No.

PCT/CN2014/092146

| Patent Documents referred in the Report | Publication Date | Patent Family | Publication Date |
|---|---|---|---|
| CN 102164596 A | 24 August 2011 | CA 2736866 A1 | 06 May 2010 |
| | | KR 20110058902 A | 01 June 2011 |
| | | EP 2340821 A1 | 06 July 2011 |
| | | KR 101322259 B1 | 28 October 2013 |
| | | JP 5665544 B2 | 04 February 2015 |
| | | EP 2340821 A4 | 03 October 2012 |
| | | WO 2010050179 A1 | 06 May 2010 |
| | | CN 103800310 A | 21 May 2014 |
| | | US 2011196033 A1 | 11 August 2011 |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• *World Health Organization (WHO),* 1998